# EUROPEAN PATENT SPECIFICATION

(11) **EP 2 588 463 B1**
(45) Date of publication and mention of the grant of the patent: **13.08.2014**
(21) Application number: 11730265.3
(22) Date of filing: 29.06.2011
(51) Int. Cl.: C07D 233/94, A61K 31/4164, A61P 17/00

(54) **METRONIDAZOLE ESTERS FOR TREATING ROSACEA**
METRONIDAZOESTER ZUR BEHANDLUNG VON ROSACEA
ESTER DE MÉTRONIDAZOLE POUR LE TRAITEMENT DE LA ROSACÉE

(30) Priority: 29.06.2010 FR 1055242
(43) Date of publication of application: 08.05.2013
(73) Proprietor: Galderma Research & Development, 06410 Biot (FR)
(72) Inventor: BOITEAU, Jean-Guy, F-06560 Valbonne (FR); LINGET, Jean-Michel, F-67230 Benfeld (FR)
(74) Representative: Tezier Herman, Béatrice
(86) International application number: PCT/EP2011/060924
(87) International publication number: WO 2012/001054

(56) References cited:
- WO-A2-02/074290
- DUBEY, S., JAIN, V., PREETHI, G.B.: "Evaluation of lipophilicity, antimicrobial activity and mutagenicity of some novel ester prodrugs of metronidazole", INDIAN JOURNAL OF CHEMISTRY, vol. 48B, 2009, pages 1571-1576, XP009139409,
- KORTING H C ET AL: "Current topical and systemic approaches to treatment of rosacea", JEADV. JOURNAL OF THE EUROPEAN ACADEMY OF DERMATOLOGY AND VENEREOLOGY, ELSEVIER SCIENCE PUBLISHERS, AMSTERDAM, NL LNKD- DOI:10.1111/J.1468-3083.2009.03167.X, vol. 23, no. 8, 1 August 2009 (2009-08-01) , pages 876-882, XP002591723, ISSN: 0926-9959 [retrieved on 2009-06-08]
- SOBOTTKA, A.: "Rosazea 2009", DER HAUTARZT, vol. 12, 2009, pages 999-1009, XP19766471,

## Description

The present invention relates to the use of a compound of formula (I) as a medicament, especially in the treatment and/or prevention of rosacea and in the treatment and/or prevention of inflammatory pathologies.

Rosacea is a progressive chronic common inflammatory dermatosis associated with vascular relaxation. It mainly affects the central part of the face and is characterized by reddening of the face or hot flushes, facial erythema, papules, pustules, telangiectasia and occasionally ocular lesions known as ocular rosacea. In serious cases, especially in men, the soft tissue of the nose may swell and produce a bulbous swelling known as rhinophyma. Rosacea develops over several years via episodes that are worsened by various stimuli such as temperature variations, alcohol, spices, exposure to sunlight, or emotions.

Rosacea is classified into four subtypes as a function of various clinical characteristics (Wilkin J. et al., JAAD, 2002, 46: 584-587).

The primary characteristics (histamine flushes, persistent erythema, papules and pustules, and telangiectasia) and secondary characteristics (burning or stinging sensation, plaques, dry appearance of the skin, oedema, ocular manifestations, phymatous changes) of rosacea are often observed in combination. The most common modes of exteriorization or combinations of signs are temporarily regrouped into specific subtypes, which are described below. Each category comprises the minimum number of signs that are sufficient to make a diagnosis of the corresponding subtype (although the modes of exteriorization are not necessarily limited to these signs), and it is possible that patients simultaneously present characteristics suggesting more than one subtype of rosacea.

### Subtype 1: Erythematotelangiectasic rosacea

Erythematotelangiectasic rosacea is characterized mainly by histamine flushes and persistent central facial erythema. The presence of telangiectasias is common, but not essential to the diagnosis of this subtype. A central facial oedema, burning and stinging sensations, and redness or desquamation are also occasionally observed. History of histamine flushes alone are common in the case of patients suffering from erythematotelangiectasic rosacea.

### Subtype 2: Papulopustular rosacea

Papulopustular rosacea is characterized by persistent central facial erythema and by transient papules and/or pustules distributed in the centre of the face. However, the papules and pustules may also affect the peri-orificial regions (i.e. the perioral, perinasal or periocular areas). The papulopustular subtype resembles common acne, but comedones are absent. Rosacea and acne may coexist, and, besides the papules and pustules resembling rosacea, the patients concerned will also possibly have comedones. Patients suffering from papulopustular rosacea occasionally complain of burning and stinging sensations.
This subtype is often observed before or at the same time as subtype 1 (including the presence of telangiectasias). The telangiectasias risk being masked by the persistent erythema and the papules or pustules.

### Subtype 3: Phymatous rosacea

Phymatous rosacea is manifested by thickening of the skin, nodules with an irregular surface and tumefaction. Rhinophyma is the commonest presentation, but phymatous rosacea may affect other regions, including the chin, the forehead, the cheeks and the ears. In the case of patients suffering from this subtype, the presence of enlarged and prominent follicular apertures is occasionally reported in the affected region, as are telangiectasias.
This subtype is often observed before or at the same time as subtype 1 or 2 (including the presence of persistent erythema, telangiectasias, papules and pustules). In the case of rhinophyma, these additional stigmata risk being particularly pronounced in the nasal region.

### Subtype 4: Ocular rosacea (or ophthalmic rosacea)

The diagnosis of ocular rosacea must be envisaged when a patient has one or more of the following ocular signs and symptoms: teary or bloodshot appearance (interpalpebral conjunctival hyperaemia), sensation of presence of a foreign body, of burning or stinging, dryness, itching, photosensitivity, blurred vision, telangiectasias of the conjunctiva and of the edge of the eyelid, or erythema of the eyelid and periocular erythema. Blepharitis, conjunctivitis and irregularity of the edges of the eyelid are other signs that may be detected. A chalazion or a chronic staphylococcic infection manifested by a stye and whose cause is a dysfunction of the meibomian glands is a frequent sign of ocular affection related to rosacea. Some patients complain of a reduction in visual acuity, which is due to corneal complications (punctuate keratitis, corneal infiltrates/corneal ulcers or marginal keratitis). By itself, the treatment of cutaneous rosacea may be without effect on the risk of lowering the visual acuity associated with ocular rosacea, and an ophthalmological approach will possibly be required.

Finally, other rarer forms of rosacea exist (variants), in particular granulomatous rosacea.

The diagnosis of ocular rosacea is most often made when cutaneous signs and symptoms are also detected. However, it is not necessary for cutaneous signs and symptoms to be present in order to make the diagnosis, and small-scale studies suggest that up to 20% of patients suffering from ocular rosacea may develop ocular signs and symptoms before cutaneous manifestations appear. Cutaneous lesions are the first to appear in the case of about half of these patients, and manifestations of the two types occur simultaneously in a minority of them.

Rosacea generally occurs between the ages of 25 and 70, and is much more common in people with fair complexion. It more particularly affects women, although this complaint is generally more severe in the case of men.

The pathogenesis of rosacea is poorly understood, and may involve several factors. These are, for example, vascular factors (abnormal vascular reactivity), immune factors, or alternatively exogenous factors such as the presence of follicular microorganisms such as bacteria and *Demodex folliculorum* mites (Diamantis S. & Waldorf H.A., J. Drug Dermatol., 2006, 5: 8-12; Wilkin J.K., Arch. Dermatol., 1994, 130: 359-362; Buechner S.A., Dermatology, 2005, 210: 100-108).

Moreover, studies, especially clinical studies, tend to suggest that rosacea is an inflammatory pathology (McKeage et al., Am. J. Clin. Dermatol. 2010; 11 (3): 217-22).

Conventionally, rosacea is treated orally or topically. Among the agents having a marketing authorization for the "rosacea" indication are topical metronidazole and oral doxycycline (Cribier B., La rosacée, Masson-Eticom, Paris, 2002).

Long-term oral treatments with tetracycline derivatives are problematic for many reasons, in particular on account of their significant side effects. The oral administration of tetracyclines, especially doxycycline, may induce photosensitivity, or even phototoxicity at and above 100 mg/day (Layton A.M., Cunliffe W.J. Phototoxic eruptions due to doxycycline-a dose-related phenomenon. Clin. Exp. Dermatol. 1993; 18:425-427), or alternatively gastrointestinal disorders (Maibach H. Second-generation tetracyclines, a dermatologic overview: clinical uses and pharmacology. Cutis. 1991; 48:411-417).

In addition, these treatments do not make it possible to effectively treat and/or prevent all of the symptoms associated with rosacea. Considering the chronic nature of rosacea, with a typical profile of remission and exacerbation, an ideal treatment requires use that may be prolonged, in a safe and effective manner.

Patent application WO 02/74290 describes the use of at least one non-steroidal anti-inflammatory drug (NSAID) for treating rosacea. This compound may especially be piroxicam, aspirin, ibufenac or naproxen. It may optionally be used in combination with a nitroimidazole. The simultaneous use of an NSAID and of a nitroimidazole has, however, appreciable side effects, especially gastrointestinal and renal effects associated with the use of metronidazole as nitroimidazole (D.I. Edwards, Br. J. Vener. Dis. 1980; 56: 285-290), or ulcers associated with the use of an NSAID (C.J. Hawkey, J. Rheumatology, 2002; 29: 4; 650-652).

There is thus a need for active agents that are effective for treating rosacea, which can be used for long periods, and which have the least possible side effects.

The aim of the present invention is thus to propose an effective treatment for rosacea, which especially reduces the side effects for the patient. Preferably, this treatment is performed topically, which considerably reduces any systemic side effect.

One subject of the present invention is thus a compound chosen from the compound of formula (I) below: 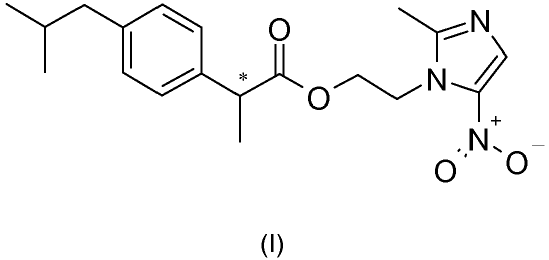 enantiomers thereof and pharmaceutically acceptable salts thereof, for its use as a medicament.

The compound of formula (I) has the chemical name 2-(2-methyl-5-nitroimidazol-1-yl)ethyl 2-(4-isobutylphenyl)propionate.
This compound contains an ester function, which is specifically cleaved in keratinocytes, as is demonstrated in Example 2. For comparative purposes, other metronidazole esters with an NSAID, for instance indomethacin, niflumic acid, diflunisal or ketorolac esters, were prepared and tested on keratinocyte cultures. All these compounds are stable in the presence of keratinocytes, unlike the compound of formula (I) according to the invention.
This particular instability of the compound of formula (I), enantiomers thereof and pharmaceutically acceptable salts thereof is thus surprising and unexpected. Without wishing to be bound by any theory, it is quite likely that this particular surprising instability of the compound of formula (I), enantiomers thereof and pharmaceutically acceptable salts thereof makes it possible to obtain the anti-inflammatory activity and the anti-rosacea activity once the compound of formula (I), enantiomers thereof or pharmaceutically acceptable salts thereof has (have) penetrated the skin and become hydrolysed on contact with the keratinocytes.

A subject of the present invention is also a compound chosen from the compound of formula (I), enantiomers thereof and pharmaceutically acceptable salts thereof, for its use in the treatment and/or prevention of rosacea.

A subject of the present invention is also a compound chosen from the compound of formula (I), enantiomers thereof and pharmaceutically acceptable salts thereof, for its use in the prevention and/or treatment of inflammatory pathologies.

A subject of the present invention is also the use of at least one compound chosen from the compound of formula (I), enantiomers thereof and pharmaceutically acceptable salts thereof, for preparing a medicament for treating and/or preventing rosacea.

Finally, a subject of the invention is the S enantiomer of the compound of formula (I) (or 2-(2-methyl-5-nitroimidazol-1-yl)ethyl (S)-2-(4-isobutylphenyl)propionate) and the pharmaceutically acceptable salts thereof.

The term "salts of the compound of formula (I) according to the invention or salts of enantiomers thereof" means salts of this compound or of enantiomers thereof with a pharmaceutically acceptable acid.
The pharmaceutically acceptable acid is especially:
- a pharmaceutically acceptable inorganic acid, for instance hydrochloric acid, sulfuric acid, phosphoric acid, nitric acid or hydrobromic acid;
- or a pharmaceutically acceptable organic acid, for instance acetic acid, tartaric acid, maleic acid, hydroxymaleic acid, fumaric acid, citric acid, lactic acid, mucic acid, gluconic acid, benzoic acid, succinic acid, oxalic acid, phenylacetic acid, methanesulfonic acid, toluenesulfonic acid, benzenesulfonic acid, salicylic acid, aspartic acid, glutamic acid and ascorbic acid.
Preferably, the salts of the compound of formula (I) are chosen from the hydrochloride, the citrate, the salicylate and the benzoate of the compound of formula (I).

The carbon atom located between the benzene ring and the -COO- group (marked with an asterisk in formula (I)) is asymmetric; the molecule is thus chiral. Thus, the term "enantiomers of the compound of formula (I)" means the R and S enantiomers of this compound. The S enantiomer is the preferred form. The S enantiomer of the compound of formula (I) is thus 2-(2-methyl-5-nitroimidazol-1-yl)ethyl (S)-2-(4-isobutylphenyl)propionate.
Preferably, the salts of the S enantiomer of the compound of formula (I) are chosen from the hydrochloride, the citrate, the salicylate and the benzoate of the said S enantiomer (i.e. 2-(2-methyl-5-nitroimidazol-1-yl)ethyl (S)-2-(4-isobutylphenyl)propionate hydrochloride, 2-(2-methyl-5-nitroimidazol-1-yl)ethyl (S)-2-(4-isobutylphenyl)propionate citrate, 2-(2-methyl-5-nitroimidazol-1-yl)ethyl (S)-2-(4-isobutylphenyl)propionate salicylate and 2-(2-methyl-5-nitroimidazol-1-yl)ethyl (S)-2-(4-isobutylphenyl)propionate benzoate.

The term "treatment" or "treating" rosacea means reducing and/or inhibiting the development of rosacea and/or of the symptoms thereof.
The term "prevention" or "preventing" rosacea means reducing and/or avoiding the appearance of the symptoms of rosacea.
The term "inflammatory pathologies" especially means cutaneous inflammatory pathologies, such as psoriasis, atopic dermatitis, acne or eczema.

The term "compound according to the invention" means, indiscriminantly, the compound of formula (I), an enantiomer thereof and/or a pharmaceutically acceptable salt thereof.

More preferentially, the compound according to the invention is the S enantiomer of the compound of formula (I) or 2-(2-methyl-5-nitroimidazol-1-yl)ethyl (S)-2-(4-isobutylphenyl)propionate of structure corresponding to formula (Ia):

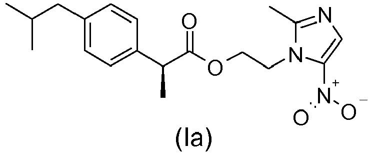

The compound according to the invention in racemic form may be prepared as indicated in the publication R.C. Prasad et al., Indian Journal of Chemistry, 1990, 29B(11), 1034. The enantiomers may themselves be separated according to processes that are known in the art, such as chiral HPLC. In particular, the S enantiomer may be prepared starting with ibuprofen in S configuration, as indicated in Example 1.

The compound chosen from the compound of formula (I), enantiomers thereof and pharmaceutically acceptable salts thereof may thus be formulated in pharmaceutical compositions for human use. The said compositions comprise, in a pharmaceutically acceptable medium, at least one compound chosen from the compound of formula (I), enantiomers thereof and pharmaceutically acceptable salts thereof.
The term "pharmaceutically acceptable medium" means a medium that is compatible with the skin, mucous membranes and the integuments.

The pharmaceutical composition that may be used according to the invention may be administered topically, parenterally or orally.
Preferably, the compound chosen from the compound of formula (I), enantiomers thereof and pharmaceutically acceptable salts thereof is present in a pharmaceutical composition for topical application.
The term "topical application" means application to the skin, mucous membranes and/or the integuments.

The composition according to the invention comprises from 0.001 % to 10% by weight of compound(s) according to the invention relative to the total weight of the composition. Preferentially, the composition according to the invention contains from 0.1% to 5% by weight of compound(s) according to the invention relative to the total weight of the composition.

The topical pharmaceutical composition may be in liquid, pasty or solid form, and more particularly in the form of an ointment, a cream, a milk, a pomade, a powder, an impregnated pad, a syndet, a wipe, a solution, a gel, a spray, a mousse, a suspension, a lotion, a stick, a shampoo or a washing base. It may also be in the form of a suspension of microspheres or nanospheres or lipid or polymer vesicles or a polymer patch and a hydrogel allowing controlled release. This pharmaceutical composition for topical application may be in anhydrous form, in aqueous form or in the form of an emulsion.

In one preferred variant of the invention, the pharmaceutical composition for topical application is in the form of a solution, a gel or an emulsion.

Such pharmaceutical compositions may be manufactured according to processes that are well known to those skilled in the art.

Various examples of preparation and use of the compounds according to the invention will now be given, for illustrative purposes and with no limiting nature.

### EXAMPLES

### Example 1: Synthesis of 2-(2-methyl-5-nitroimidazol-1-yl)ethyl (S)-2-(4-isobutylphenyl)propionate

To a solution of (S)-ibuprofen (5 g, 24.23 mmol) and metronidazole (4.15 g, 24.23 mmol) in 50 mL of dichloromethane are successively added 100 mg of DMAP (4-dimethylaminopyridine) and then 5.1 g (26.6 mmol) of EDC (1-ethyl-3-(3-dimethylaminopropyl)carbodiimide). The reaction mixture is stirred for 4 hours at room temperature and then quenched with 100 mL of water and extracted with 100 mL of dichloromethane. The organic phase is washed with 2x100 mL of 5% citric acid solution and then with 100 mL of water. The organic phase is dried over magnesium sulfate and then evaporated under reduced pressure. The residue is crystallized from pentane.
7.4 g of 2-(2-methyl-5-nitroimidazol-1-yl)ethyl (S)-2-(4-isobutylphenyl)propionate are obtained.
Yield = 84%.
**¹H NMR (CDCl₃):** 7.94 (s, 1 H); 7.10 (s, 4H); 4.56-4.39 (m, 4H); 3.62 (q, 1 H, J= 7 Hz); 2.47 (d, 2H, J= 7 Hz); 2.21 (s, 3H); 1.87 (m, 1 H, J= 6.7 Hz); 1.46 (d, 3H, J= 7 Hz); 0.95 (d, 6H, J= 6.6 Hz).
**¹³C NMR (CDCl₃):** 174.2; 151.0; 137.0; 133.0; 129.6; 127.0; 62.8; 45.0; 45.0; 45.1; 30.2; 22.4; 22.4; 18.4; 14.0.
NB: In order to obtain the compound of formula (I) according to the invention in racemic form, the process described above is used replacing the S-ibuprofen with racemic ibuprofen.
Each enantiomer of the compound of formula (I) is identified by chiral HPLC under the following conditions:

| | |
|---|---|
| Column IC 250x4.6 mm 5 µm | |
| Route A Heptane | 75% + 0.1 % TFA |
| Route B Isopropanol | 25% + 0.1 % TFA |
| Flow rate: 1.2 ml/min | |
| (S): 8.2 minutes / (R): 10 minutes | |

### Example 2: Stabilities evaluated on keratinocyte cultures

The stability on keratinocyte cultures is evaluated using human neonatal keratinocytes (Cell'N Tech) cultured in a 75 cm² flask and detached at 90-100% of confluence with vegetable trypsin (trypLE GIBCO). The compound of formula (I) was tested at 2 µM in the medium CNT-057 (Cell'N Tech) supplemented with 0.1% pluronic acid in 24-well plates. The final DMSO concentration is 0.1%. Degradation kinetics are performed with a Tecan EVO robot over 24 hours. The samples taken are then assayed by LC/MS/MS (Micromass) in comparison with a calibration range of the test product, prepared under the same conditions as the samples (1/3 culture medium and 2/3 methanol). The chromatographic conditions are optimized for each product. An assay of the appearance of metronidazole is also performed using these same samples.
The half-life time (t_{1/2}) of the racemic compound of formula (I), (i.e. 2-(2-methyl-5-nitroimidazol-1-yl)ethyl 2-(4-isobutylphenyl)propionate) is 3 hours.
The half-life time (t_{1/2}) of the S enantiomer of the compound of formula (I), (i.e. 2-(2-methyl-5-nitroimidazol-1-yl)ethyl (S)-2-(4-isobutylphenyl)propionate) is 20 hours.

For comparative purposes, the metronidazole esters with indomethacin, niflumic acid, diflunisal or ketorolac do not become hydrolysed on the keratinocyte cultures.

### Example 3: Anti-inflammatory activity

### a) Model of acute inflammation on mouse ear: arachidonic acid-induced oedema

The anti-inflammatory activities of the compound of formula (I) in racemic form, of the S enantiomer of the compound of formula (I), of metronidazole and of ibuprofen were measured in an in vivo test performed on a mouse model. This model consists in inducing inflammation of the mouse ear with a single application of arachidonic acid (AA). Female Balb/c ByJlco mice are used. The oedema is induced by applying to the mouse's ear 20µL of AA dissolved to 4% in a 1/1 THF/methanol mixture. The anti-inflammatory activities of the S enantiomer of the compound of formula (I), of the compound of formula (I) in racemic form, of metronidazole and of ibuprofen were evaluated after topical application onto the inner face of the mouse's ear of 20µL of a solution of the compound in a 1/1 THF/methanol mixture containing 4% AA.

Solutions containing 0.01%; 0.03%; 0.1%; 0.3%; 1% and 2% of the test compound are thus prepared. The percentages correspond to the weight of the test compound per volume of solution. The oedema is evaluated, 1 hour after application, by measuring the thickness of the ear using an Oditest® micrometer. The oedema induced by AA alone is evaluated, 1 hour after application, by measuring the thickness of the ear using an Oditest® micrometer. It is evaluated relative to a group comprising only the vehicle (1/1 THF/methanol).
The inhibition of the oedema is expressed as a percentage of reduction, by the test molecule and at the test concentration, of the oedema induced by AA alone.
The IC₅₀ corresponds to the concentration (or dose) at which 50% inhibition of the oedema induced by AA alone is observed.

The results are as follows:
- metronidazole has no anti-inflammatory activity in this model;
- the S enantiomer of the compound of formula (I), namely 2-(2-methyl-5-nitroimidazol-1-yl)ethyl (S)-2-(4-isobutylphenyl)propionate, inhibits by 49%, at a dose of 1 %, the oedema induced by AA alone.

### b) Model of acute inflammation on mouse ear: oedema induced by TPA (phorbol 12-myristate-13-acetate)

The anti-inflammatory activities of the compound of formula (I) in racemic form, of the S enantiomer of the compound of formula (I), of metronidazole and of ibuprofen were measured in an in vivo test performed on a mouse model.
The model used is the model of mouse ear inflammation induced by a single application of TPA (phorbol 12-myristate-13-acetate). Female Balb/c ByJlco mice are used.

The oedema is induced by applying to the ear 20 µl of 0.01% TPA dissolved in ethanol.

The racemic compound of formula (I) is dissolved, at the desired concentration, in the 0.01% TPA solution and thus applied to a group of mice at the same time as the TPA.
The S enantiomer of the compound of formula (I) is dissolved, at the desired concentration, in the 0.01 % TPA solution and thus applied to another group of mice at the same time as the TPA.
Similarly, ibuprofen (racemic) is dissolved, at the desired concentration, in the 0.01% TPA solution and is applied to another group of mice at the same time as the TPA. Finally, metronidazole is dissolved, at the desired concentration, in the 0.01% TPA solution and is applied to another group of mice at the same time as the TPA.

The oedema is evaluated, 6 hours after application of the test product, by measuring the thickness of the ear using an Oditest® micrometer.
The TPA-induced oedema is evaluated relative to the ethanol vehicle group. The inhibition, with the racemic compound of formula (I), with the S enantiomer of the compound of formula (I), with ibuprofen (racemic) or with metronidazole, of the oedema induced by TPA alone is expressed by the IC₅₀.
The IC₅₀ corresponds to the concentration (or dose) at which 50% inhibition of the oedema induced by TPA alone is observed.

The results are as follows:
metronidazole has no anti-inflammatory activity in this model;
The compound of formula (I) in racemic form, namely 2-(2-methyl-5-nitroimidazol-1-yl)ethyl 2-(4-isobutylphenyl)propionate, itself has an IC₅₀ equal to 0.28%.
This compound thus has noteworthy anti-inflammatory activity.

## Claims

1. Compound chosen from the compound of formula (I) below: 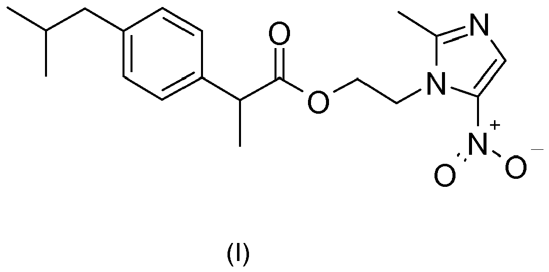 enantiomers thereof and pharmaceutically acceptable salts thereof, for its use as a medicament.

2. Compound according to Claim 1, for its use in the prevention and/or treatment of rosacea.

3. Compound according to Claim 1, for its use in the prevention and/or treatment of inflammatory pathologies.

4. Compound according to Claims 1 to 3, **characterized in that** the salt of the compound of formula (I) or of an enantiomer thereof is chosen from the salts of this compound or of an enantiomer thereof with a pharmaceutically acceptable acid.

5. Compound according to Claim 4, **characterized in that** the pharmaceutically acceptable acid is chosen from:
- pharmaceutically acceptable inorganic acids, for instance hydrochloric acid, sulfuric acid, phosphoric acid, nitric acid or hydrobromic acid;
- and pharmaceutically acceptable organic acids, for instance acetic acid, tartaric acid, maleic acid, hydroxymaleic acid, fumaric acid, citric acid, lactic acid, mucic acid, gluconic acid, benzoic acid, succinic acid, oxalic acid, phenylacetic acid, methanesulfonic acid, toluenesulfonic acid, benzenesulfonic acid, salicylic acid, aspartic acid, glutamic acid and ascorbic acid.

6. Compound according to one of Claims 1 to 5, **characterized in that** it is chosen from the compound of formula (I), the hydrochloride of the compound of formula (I), the citrate of the compound of formula (I), the salicylate of the compound of formula (I), the benzoate of the compound of formula (I), and the S enantiomers of these compounds.

7. Compound according to one of Claims 1 to 6, **characterized in that** it is 2-(2-methyl-5-nitroimidazol-1-yl)ethyl (S)-2-(4-isobutylphenyl)propionate.

8. Compound according to one of Claims 1 to 7, **characterized in that** it is in a pharmaceutical composition for topical application.

9. Compound according to Claim 8, **characterized in that** the pharmaceutical composition is in the form of a solution, a gel or an emulsion.

10. Compound according to one of Claims 1 to 9, **characterized in that** it is present in an amount of between 0.001 % and 10% by weight relative to the total weight of the composition.

11. 2-(2-Methyl-5-nitroimidazol-1-yl)ethyl (S)-2-(4-isobutylphenyl)propionate of structure corresponding to formula (Ia): 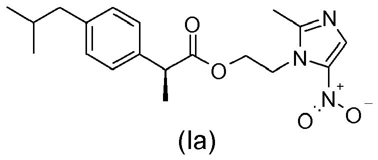 and pharmaceutically acceptable salts thereof.

## Patentansprüche

1. Verbindung ausgewählt aus der Verbindung der Formel (I) unten: 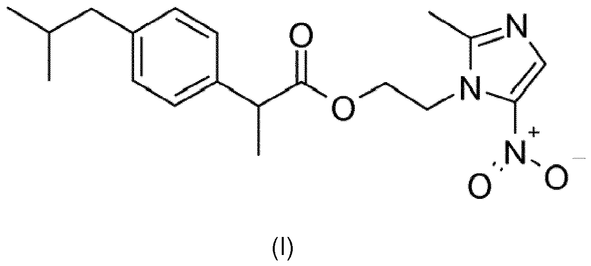 Enantiomere davon und pharmazeutisch annehmbare Salze davon, für ihre Verwendung als ein Medikament.

2. Verbindung nach Anspruch 1, für ihre Verwendung bei der Vermeidung und/oder Behandlung von Rosacea.

3. Verbindung nach Anspruch 1, für ihre Verwendung bei der Vermeidung und/oder Behandlung von entzündlichen Pathologien.

4. Verbindung nach Ansprüchen 1 bis 3, **dadurch gekennzeichnet, dass** das Salz der Verbindung der Formel (I) oder eines Enantiomers davon ausgewählt ist aus den Salzen dieser Verbindung oder eines Enantiomers davon mit einer pharmazeutisch annehmbaren Säure.

5. Verbindung nach Anspruch 4, **dadurch gekennzeichnet, dass** die pharmazeutisch annehmbare Säure ausgewählt ist aus:
- pharmazeutisch annehmbaren anorganischen Säuren, zum Beispiel Salzsäure, Schwefelsäure, Phosphorsäure, Salpetersäure oder Bromwasserstoffsäure;
- und pharmazeutisch annehmbaren organischen Säuren, zum Beispiel Essigsäure, Weinsäure, Maleinsäure, Hydroxymaleinsäure, Fumarsäure, Zitronensäure, Milchsäure, Schleimsäure, Gluconsäure, Benzoesäure, Bernsteinsäure, Oxalsäure, Phenylessigsäure, Methansulfonsäure, Toluolsulfonsäure, Benzolsulfonsäure, Salicylsäure, Asparaginsäure, Glutaminsäure und Ascorbinsäure.

6. Verbindung nach einem der Ansprüche 1 bis 5, **dadurch gekennzeichnet, dass** sie ausgewählt ist aus der Verbindung der Formel (I), dem Hydrochlorid der Verbindung der Formel (I), dem Citrat der Verbindung der Formel (I), dem Salicylat der Verbindung der Formel (I), dem Benzoat der Verbindung der Formel (I) und den S-Enantiomeren dieser Verbindungen.

7. Verbindung nach einem der Ansprüche 1 bis 6, **dadurch gekennzeichnet, dass** sie 2-(2-Methyl-5-nitroimidazol-1-yl)-ethyl-(S)-2-(4-isobutylphenyl)-propionat ist.

8. Verbindung nach einem der Ansprüche 1 bis 7, **dadurch gekennzeichnet, dass** sie in einer pharmazeutischen Zusammensetzung zur topischen Anwendung vorliegt.

9. Verbindung nach Anspruch 8, **dadurch gekennzeichnet, dass** die pharmazeutische Zusammensetzung in Form einer Lösung, eines Gels oder einer Emulsion vorliegt.

10. Verbindung nach einem der Ansprüche 1 bis 9, **dadurch gekennzeichnet, dass** sie in einer Menge von zwischen 0,001 Gewichts-% und 10 Gewichts-%, bezogen auf das Gesamtgewicht der Zusammensetzung, enthalten ist.

11. 2-(2-Methyl-5-nitroimidazol-1-yl)-ethyl-(S)-2-(4-isobutylphenyl)-propionat der Struktur, die der Formel (Ia) entspricht: 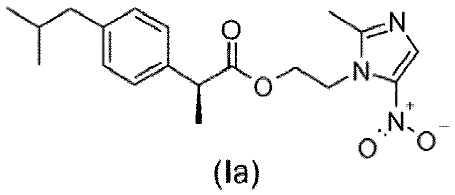 und pharmazeutisch annehmbare Salze davon.

## Revendications

1. Composé choisi parmi le composé de formule (I) ci-dessous : 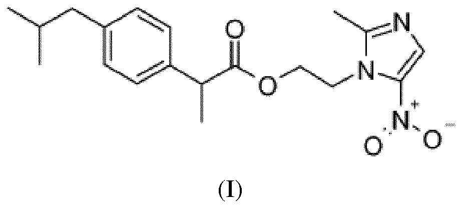 des énantiomères de celui-ci et des sels pharmaceutiquement acceptable de celui-ci, pour son utilisation en tant que médicament.

2. Composé selon la revendication 1, pour son utilisation dans la prévention et/ou le traitement de la rosacée.

3. Composé selon la revendication 1, pour son utilisation dans la prévention et/ou le traitement de pathologies inflammatoires.

4. Composé selon les revendications 1 à 3, **caractérisé en ce que** le sel du composé de formule (I) ou d'un énantiomère de celui-ci est choisi parmi les sels de ce composé ou d'un énantiomère de celui-ci avec un acide pharmaceutiquement acceptable.

5. Composé selon la revendication 4, **caractérisé en ce que** l'acide pharmaceutiquement acceptable est choisi parmi :
- des acides inorganiques pharmaceutiquement acceptables, par exemple l'acide chlorhydrique, l'acide sulfurique, l'acide phosphorique, l'acide nitrique ou l'acide bromhydrique ;
- et des acides organiques pharmaceutiquement acceptables, par exemple l'acide acétique, l'acide tartrique, l'acide maléique, l'acide hydroxymaléique, l'acide fumarique, l'acide citrique, l'acide lactique, l'acide mucique, l'acide gluconique, l'acide benzoïque, l'acide succinique, l'acide oxalique, l'acide phénylacétique, l'acide méthylsulfonique, l'acide toluènesulfonique, l'acide benzènesulfonique, l'acide salicylique, l'acide aspartique, l'acide glutamique et l'acide ascorbique.

6. Composé selon une des revendications 1 à 5, **caractérisé en ce qu'**il est choisi parmi le composé de formule (I), le chlorhydrate du composé de formule (I), le citrate du composé de formule (I), le salicylate du composé de formule (I), le benzoate du composé de formule (I), et les énantiomères S de ces composés.

7. Composé selon l'une des revendications 1 à 6, **caractérisé en ce qu'**il est le propionate de 2-(2-méthyl-5-nitroimidazol-1-yl)éthyl (S)-2-(4-isobutylphényl).

8. Composé selon l'une des revendications 1 à 7, **caractérisé en ce qu'**il est dans une composition pharmaceutique pour une administration topique.

9. Composé selon la revendication 8, **caractérisé en ce que** la composition pharmaceutique est sous la forme d'une solution, d'un gel ou d'une émulsion.

10. Composé selon l'une des revendications 1 à 9, **caractérisé en ce qu'**il est présent dans une quantité comprise entre 0.001% et 10% en poids par rapport au poids total de la composition.

11. Propionate de 2-(2-méthyl-5-nitroimidazol-1-yl)éthyl (S)-2-(4-isobutylphényl) de structure correspond à la formule (Ia) : 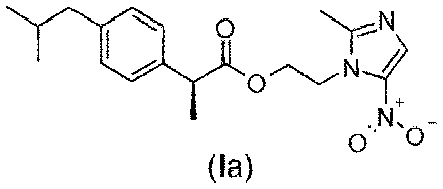 et des sels pharmaceutiquement acceptables de celui-ci.
